# EUROPÄISCHE PATENTSCHRIFT

(11) **EP 1 345 612 B1**
(45) Veröffentlichungstag und Bekanntmachung des Hinweises auf die Patenterteilung: **16.03.2005**
(21) Anmeldenummer: 01270336.9
(22) Anmeldetag: 05.12.2001
(51) Int. Cl.: A61K 31/715, A61K 31/70, A61P 19/02, A61P 19/04, A61P 17/02

(54) **VERWENDUNG VON NIEDERMOLEKULAREN HEPARIN ZUR BEHANDLUNG VON OSTEOARTHROSE**
USE OF LOW MOLECULAR HEPARIN FOR TREATING OSTEOARTHRITIS
UTILISATION D'HEPARINE DE FAIBLE MASSE MOLECULAIRE POUR LE TRAITEMENT DE L'OSTEOARTHROSE

(30) Priorität: 16.12.2000 DE 10063006
(43) Veröffentlichungstag der Anmeldung: 24.09.2003
(73) Patentinhaber: Aventis Pharma Deutschland GmbH, 65929 Frankfurt am Main (DE)
(72) Erfinder: KERN, Christopher, 67480 Edenkoben (DE); HOERBER, Christine, 55118 Mainz (DE); BARTNIK, Eckart, 65205 Wiesbaden-Delkenheim (DE); HAUS-SEUFFERT, Philipp, 80539 München (DE)
(86) Internationale Anmeldenummer: PCT/EP2001/014261
(87) Internationale Veröffentlichungsnummer: WO 2002/047696

(56) Entgegenhaltungen:
- WO-A-00/40225
- WO-A-01/29055
- WO-A-01/91729
- WO-A-02/32406
- WO-A-92/19249
- US-A- 5 648 359
- US-A- 5 686 431

## Beschreibung

Niedermolekulare Heparine (Low Molecular Weight Heparin; LMWH) haben ein durchschnittliches Molekulargewicht von etwa 3000 bis etwa 10000. Kommerziell erhältliche LMWHe sind beispielsweise:
Enoxaparin (Clexane®, Klexane®, Lovenox®), das zur Behandlung von Thrombosen eingesetzt (US 5,389,618) wird. Enoxaparin-Na ist das Natrium-Salz von niedermolekularem Heparin, das durch alkalische Depolymerisation des Benzylesterderivates von Heparin aus Schweine-Intestinal-Mucosa gewonnen wird. Die Hauptmenge der Komponenten trägt eine 4-enopyranose-Uronat-Struktur am Nicht-reduzierenden-Ende ihrer Kette.
Die durchschnittliche Molekülmasse beträgt etwa 4500 Dalton.
Der prozentuale Anteil von Molekülen kleiner 2000 Dalton liegt zwischen 12% und 20%. Die Massenprozent-Anteil der Ketten einer Größe zwischen 2000 und 8000 Dalton liegt zwischen 68% und 88% in Bezug auf den European Pharmacopoeia calibration reference Standard für niedermolekulare Heparine. Der Sulfatierungsgrad liegt bei 2 pro Disaccharid-Einheit. Die Enoxaparin-Polysaccharid-Kette ist wie bei Heparin aus alternierenden Einheiten von sulfatierten Glucosaminen und Uron-Säuren, die durch glycosidische Bindungen verknüpft sind, zusammengesetzt. Die Struktur unterscheidet sich von Heparin beispielsweise dadurch, dass durch den Depolymerisierungsprozess eine Doppelbindung am Nicht-reduzierenden-Ende der Kette entsteht.
Enoxaparin kann von Heparin unterschieden werden durch, UV-Spectroskopie und durch das ¹³C nuclear magnetic resonance Spektrum, welche die Doppelbindung im terminalen Ring zeigen, und durch high performance size exclusion chromatography. Nardroparin (Fraxiparin®) mit einer durchschnitlliche molekularen Masse von 4300 ± 700 Dalton (Da), wobei Komponenten mit einer molekularen Masse von < 2000 Da (<15 %), 2000 bis 4000 Da (45 % ± 10 %) und 2000 bis 8000 Da (85 % ± 10 %) enthalten sind.
Dalteparin (Fragmin®) mit einer durchschnitlliche molekularen Masse von 6000 ± 400 Da, wobei Komponenten mit einer molekularen Masse von < 3000 Da (5 % bis 13 %) und größer als 8000 Da (15 % bis 25 %) enthalten sind.

Certroparin (Embolex®, Monoembolex ®) mit einer durchschnittliche molekularen Masse von 5200 ± 1000 Da, wobei Komponenten mit einer molekularen Masse von < 2000 Da (10 % bis 25 %) und kleiner 8000 Da (75 % bis 90 %) enthalten sind. Parnaparin (Fiuxum®, Minidalton®) mit einer durchschnitlliche molekularen Masse von 5000 ± 1000 Da, wobei Komponenten mit einer molekularen Masse von von < 3000 Da (20 % bis 30 %) und von 3000 Da bis 8000 Da (50 % bis 60 %).
Tinzaparin (Logiparin®) mit einer durchschnitlliche molekularen Masse von 3400 Da bis 5600 Da, wobei Komponenten mit einer molekularen Masse von < 2000 Da (2 % bis 16 %), 2000 Da bis 4000 Da (66 % ± 6 %) und größer 8000 Da (12 % bis 38 %) enthalten sind. Ferner sind bekannt Reviparin (Clivarine®) und Ardeparin/RD Heparin/RDH (Normiflo®).
Die LMWH sind beispielsweise herstellbar aus Heparin durch Ethanol Fraktionierung oder Gelfiltration, der in Heparin natürlich vorkommenden niedermolekularen Fraktionen. Heparin kann aber auch mit Hilfe von Salpetersäure, β-Elimination oder Perjodatoxidation oder durch Enzyme kontrolliert depolymerisiert und anschließend fraktioniert werden.

Im Krankheitsbild der Osteoarthrose stellt die Degradation des Aggrecans, das Hauptproteoglykan des artikulären Knorpels, ein sehr frühes und entscheidendes Ereignis dar. Der pathologische Verlust des Aggrecans, der das Hauptproteoglykan des Knorpels darstellt, beinhaltet proteolytische Spaltungen in seiner interglobulären Domäne. Aminosäuresequenzanalysen von Proteoglykanmetaboliten, isoliert aus der Synovialflüssigkeit von Patienten, die an einer Gelenkschädigung, an Osteoarthrose oder an einer entzündlichen Gelenkerkrankung leiden, zeigten, daß eine proteolytische Spaltung bevorzugt zwischen den Aminosäuren Glu³⁷³ und Ala³⁷⁴ in der interglobulären Domäne des humanen Aggrecans stattfindet (Lohmander et al. Arthritis Rheum. 36, (1993), 1214-1222). Die proteolytische Aktivität, die für diese Spaltung verantwortlich ist, wird als "Aggrecanase" bezeichnet und kann zur Superfamilie der Metalloproteinasen (MP) bzw. Matrix-Metalloproteinasen gerechnet werden.

Bei den Metalloproteinasen ist Zink im katalytisch aktiven Zentrum essentiell. MMP spalten Kollagen, Laminin, Proteoglykane, Elastin oder Gelatin unter physiologischen Bedingungen und spielen daher eine wichtige Rolle im Knochen und Bindegewebe.

Eine Vielzahl von verschiedenen Inhibitoren der MMPs sind bekannt (EP 0 606 046; WO94/28889).

Nachteil der bekannten Inhibitoren der MMPs ist häufig die mangelnde Spezifität der Hemmung für nur eine Klasse der MMPs. Daher hemmen die meisten MMP-Inhibitoren mehrere MMPs gleichzeitig.

In dem Bestreben, wirksame Verbindungen zur Behandlung von Bindegewebserkrankungen zu finden, wurde nun gefunden, dass das erfindungsgemäß eingesetzte Enoxaparin ein starker Inhibitor der Matrix-Metalloproteinasen neutrophilen Kollagenase (MMP-8), Aggrecanase, hADAMTS1 und Gelatinase A (MMP-2) ist, während das Enoxaparin im wesentlichen unwirksam bei den MMPs 1, 3, 13 und 14 ist.

Die Erfindung betrifft daher die Verwendung von niedermolekularen Heparin mit einem durchschnittlichen Molekulargewicht von etwa 3000 bis etwa 10000 aus der Reihe Enoxaparin, Nardroparin, Dalteparin, Certroparin, Pamaparin, Reviparin, Ardeparin/RD Heparin/RDH oder Tinzaparin oder Mischungen derselben zur Herstellung von Arzneimitteln zur Prophylaxe und Therapie von Erkrankungen, an deren Verlauf eine verstärkte Aktivität mindestens einer der Matrix-Metalloproteinasen neutrophilen. Kollagenase (MMP-8), Aggrecanase, hADAMTS1 und Gelatinase A (MMP-2) beteiligt ist aus der Reihe Osteoarthrosen, Spondylosen, Knorpelschwund nach Gelenktrauma oder längerer Gelenksruhigstellung nach Meniskus- oder Patellaverletzungen oder Bänderrissen oder eine Erkrankung des Bindegewebes wie Kollagenosen, Wundheilungsstörungen, oder Periodontalerkrankungen.

Bevorzugt wird Enoxaparin eingesetzt.

Enoxaparin und physiologisch verträgliche Salze von Enoxaparin sind bekannt und lassen sich beispielsweise wie in US 5,389,618 beschrieben herstellen.

Die erfindungsgemäße Verwendung der niedermolekularen Heparine erfolgt im allgemeinen parenteral. Die Applikation der niedermolekularen Heparine kann durch subkutane, intraartikuläre, intraperitoneale oder intravenöse Injektion erfolgen. Bevorzugt ist die intraartikuläre Injektion. Die rektale, orale, inhalative oder transdermale Applikation ist auch möglich.

Vorzugsweise werden die pharmazeutischen Präparate in Dosierungseinheiten hergestellt und verabreicht, wobei jede Einheit als aktiven Bestandteil eine bestimmte Dosis des niedermolekularen Heparins enthält. Bei Injektionslösungen in Ampullenform kann diese Dosis von etwa 5 µg bis zu etwa 200 mg, vorzugsweise aber von etwa 10 µg bis 40 mg, betragen.

Für die Behandlung eines erwachsenen, etwa 70 kg schweren Patienten sind Tagesdosen an Enoxaparin von etwa 10 µg bis 500 mg, bevorzugt etwa 20 mg bis 100 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Die Erfindung betrifft ferner die Verwendung von ADAMTS1 zur Herstellung eines Testkits zur Bestimmung eines Inhibitors für die Aggrecanase, der dadurch gekennzeichnet ist, dass man ADAMTS1, ein Substrat und einen Inhibitor inkubiert und die durch die Aggrecanase-Aktivität generierten Neoepitope bestimmt.

### Beispiel 1

### Wirkung von Enoxaparin auf die Aggrecanase von Schweinechondrozyten

Zur Generierung der Aggrecanase-Aktivität wurden Schweinechondrozyten für 4 Tage mit 10ng/ml mit IL-1α stimuliert (Hughes, C.E., Little,C.B., Buettner, F.H., Bartnik, E., Caterson, B., (1998) Differential expression of Aggrecanase and Matrix metalloproteinase activity in chondrocytes isolated from bovine and porcine articular cartilage. J. Biol. Chem. 273, 30576-30582). In einer 96-well-Zellkulturplatte wurden 200 µl des Aggrecanase-Aktivität haltigen Chondrocytenüberstandes mit 100µl (Zellkulturmedium / Puffer) DMEM pro well gemischt. Als Inhibitor wurden 5 µl Enoxaparin, das in entsprechender Konzentration in H₂O gelöst wurde, eine Stunde vor Substratzugabe (5 µg rAgg1mut (Büttner et al.; Biochem. J. (1998), 333, 159-165)) zugegeben. Der Ansatz wurde für 17 h bei 37°C inkubiert und anschließend in eine ELISA-Platte überführt, um die durch Aggrecanase-Aktivität generierten Neoepitope mit dem Antikörper BC-3 (Hughes et al., Biochem. J. (1995), 305, 799-804) zu detektieren (Büttner et al., Trans. Orthop. Res. Soc. (1998), 23, 916).

Die Enzymaktivität wird durch ein repräsentatives Experiment in Tabelle 1 dargestellt als BC-3 Signal (Extinktion)

**Tabelle 1**

| Verdau von rAgg1mut durch Schweine-Chondrocyten-Aggrecanase: Inhibition durch Enoxaparin | | |
|---|---|---|
| Enoxaparin (µg/ml) | mittleres BC-3 Signal n=2 | Standardabweichung |
| 0 | 1,15 | 0,031 |
| 0,0167 | 1,24 | 0,020 |
| 0,167 | 1,13 | 0,031 |
| 1,67 | 1,05 | 0,027 |
| 16,7 | 0,70 | 0,049 |
| 167 | 0,33 | 0,041 |
| 1670 | 0,44 | 0,027 |
| 16700 | 0,27 | 0,063 |
| keine Aggrecanase und kein Enoxaparin | 0,06 | 0,002 |
| keine Aggrecanase und 16700 µg/ml Enoxaparin | 0,07 | 0,019 |

Der IC₅₀ läßt sich bei diesem Versuchsansatz bei etwa 80 µg/ml Enoxaparin bestimmen. Enoxaparin inhibiert die Aggrecanase-Aktivität von Schweinechondrozyten beim Verdau des Substrates rAgg1mut.

### Beispiel 2

### Wirkung von Enoxaparin auf die Aggrecanase von humanen dedifferenzierte Chondrozyten

Zur Generierung der Aggrecanase-Aktivität wurden in einer 96-well-Zellkulturplatte 50000 humane dedifferenzierte Chondrozyten pro well für 47 h mit 0,01 ng/ml IL-1□ und 3 U TNFα in 200µl DMEM/F12 Medium (1:1) stimmuliert. In einer 96-well-Zellkulturplatte wurden 200 µl des Aggrecanase-Aktivität haltigen Chondrocytenüberstandes mit 5 µl Enoxaparin (Clexane) als Inhibitor, das in entsprechender Konzentration in H₂O gelöst wurde, eine Stunde vor Substratzugabe (2,5 µg rAgg1 mut) vermischt. Der Ansatz wurde für 4 h bei 37 °C inkubiert und anschließend in eine ELISA-Platte überführt, um die durch Aggrecanase-Aktivität generierten Neoepitope mit dem Antikörper BC-3 zu detektieren.

Die Enzymaktivität wird durch ein repräsentatives Experiment in Tabelle 2 dargestellt als BC-3 Signal (Extinktion).

**Tabelle 2**

| Enoxaparin µg/ml | Clexane 40 n=2 | Clexane 20 n=2 | Standardabweichung (Clex 40) | Standardabweichung (Clex 20) |
|---|---|---|---|---|
| 0 | 1,20 | | 0,043 | |
| 0,0167 | 1,13 | 1,05 | 0,091 | 0,017 |
| 0,167 | 1,0 | 0,97 | 0,025 | 0,044 |
| 1,67 | 0,99 | 0,92 | 0,064 | 0,009 |
| 16,7 | 0,95 | 0,87 | 0,037 | 0,048 |
| 167 | 0,72 | 0,72 | 0,043 | 0,002 |
| 1670 | 0,29 | 0,42 | 0,007 | 0,084 |
| keine Aggrecanase und kein Enoxaparin | 0,41 | | 0,016 | |
| Keine Aggrecanase und 1670 µg/ml Enoxaparin | 0,19 | 0,22 | | |

Der IC₅₀ ließt sich bei diesem Testsystem bei etwa 200 µg/ml Enoxaparin bestimmen.

Enoxaparin inhibiert die Aggrecanase-Aktivität von humanen dedifferenzierte Chondrozyten beim Verdau des Substrates rAgg1mut.

### Beispiel 3

### Wirkung von Enoxaparin auf die Aggrecanase-Aktivität von rekombinantem humanem ADAMTS1 Protein

Zur Generierung der Aggrecanase-Aktivität humaner ADAMTS1 wurden 293 Zellen mit einem ADAMTS1 Expressionsplasmid nach der Calcium-Phosphat Methode transfiziert. Das selbst konstruierte Expressionsplasmid trägt die kodierende Sequenz des humanen ADAMTS1 Gens, dem ein zusätzlich eingefügtes C-terminales FLAG-tag folgt. Das Gen steht unter der Expressions-Kontrolle des CMV-Promotors. Die Überstände der Transfektion werden über eine M2 Antikörper Agarose Säule gegeben und das über den FLAG-tag an den M2-Antikörper gebundene hADAMTS1 mit FLAG-Peptid eluiert. Das so angereinigte humane ADAMTS1 wird im Aggrecanase-Assay eingesetzt.

In einer 96-well-Zellkulturplatte wurden 10 µl Eluat mit rekombinantem humanem ADAMTS1 und 300µl DMEM mit 5 µl Enoxaparin (Clexane) als Inhibitor, das in entsprechender Konzentration in H₂O gelöst wurde, eine Stunde vor Substratzugabe (1 µg rAgg1mut) vermischt. Der Ansatz wurde für 4 h bei 37°C inkubiert und anschließend in eine ELISA-Platte überführt, um die durch Aggrecanase-Aktivität generierten Neoepitope mit dem Antikörper BC-3 (Hughes et al., Biochem. J. 305, 799-804, 1995) zu detektieren (Buettner et al., Trans. Orthop. Res. Soc. 23, 916, 1998).

Die Enzymaktivität eines repräsentativen Experiments wird in Tabelle 3 dargestellt als BC-3 Signal (Extinktion)

**Tabelle 3**

| Enoxaparin µg/ml | mittleres BC-3 Signal n=2 |
|---|---|
| 0 | 1,36 |
| 0,0167 | 1,35 |
| 0,167 | 1,34 |
| 1,67 | 1,32 |
| 16,7 | 0,87 |
| 167 | 0,27 |
| 1670 | 0,23 |
| kein hADAMTS1/ kein Enoxaparin | 0,11 |

Der IC50 läßt sich bei diesem Assay bei etwa 25 µg/ml Enoxaparin bestimmen. Enoxaparin inhibiert die Aggrecanase-Aktivität von rekombinantem humanen ADAMTS1 beim Verdau des Substrates rAgg1mut.

### Beispiel 4

### Keine Wirkung von Enoxaparin auf die katalytische Domäne von MMP-3 beim Verdau des rekombinanten Substrates rAgg1mut

In einer 96-well-Zellkulturplatte wurden 31,3 µl recombinante humane MMP-3 (katalytische Domäne: G98-P273 präpariert nach der Methode von Ye et al., Biochemistry, 31, 11231-11235, 1992) in MMP-3 Verdaupuffer (0,1 M MES; 0,1 M NaCl; 0,01 M CaCl₂; 0,5% Brij; pH 6,0) mit 5 µl Enoxaparin (Clexane) als Inhibitor, das in entsprechender Konzentration in H₂O gelöst wurde, eine Stunde vor Substratzugabe (5 µg rAgg1mut) vermischt. Der Ansatz wurde für 8 h bei 37°C inkubiert und anschließend in eine ELISA-Platte überführt, um die durch MMP-3-Aktivität generierten Neoepitope (Spaltung an den Aminosäuren N341-F342) mit dem Antikörper BC-14 (Hughes et al., Biochem. J. (1995), 305, 799-804) zu detektieren (Buettner et al., Trans. Orthop. Res. Soc. 23, 916, 1998).

Die Tabelle 4 zeigt die Ergebnisse eines repräsentativen Experiments.

**Tabelle 4**

| Enoxaparin µg/ml | mittleres BC-14 Signal n=2 | Standardabweichung |
|---|---|---|
| 0 | 0,97 | 0,044 |
| 0,0167 | 1,02 | 0,004 |
| 1,67 | 1,03 | 0,075 |
| 167 | 0,96 | 0,019 |
| 16700 | 0,94 | 0,042 |
| kein MMP-3 und kein Enoxaparin | 0,14 | 0,057 |
| kein MMP-3 und 16700 µg/ml Enoxaparin | 0,12 | 0,050 |

Enoxaparin zeigte keine Wirkung auf die katalytische Domäne von MMP-3 beim Verdau des rekombinanten Substrates rAgg1mut.

### Beispiel 5

### Darstellung und Bestimmung der enzymatischen Aktivität der Gelatinase A (MMP-2) und der Neutrophilen-Kollagenase (MMP-8).

Die beiden Enzyme Gelatinase A (MMP-2) und Neutrophile-Kollagenase (MMP-8) wurden von Roche bzw Biocon bezogen. Zur Messung der Enzymaktivität oder der Enzyminhibitorwirkung werden 10 µl Enzym-haltige Pufferlösung mit 10 µl H₂O das gegebenenfalls den Enzyminhibitor enthält, für 15 Minuten inkubiert. MMP-2 (20 mUnit) oder MMP-8 (20ng) werden nach der von Knight beschriebenen Methode (Knight et al. (1992) FEBS letters 296,263) mit 10 µl einer 10%igen (v/v) wäßrigen Dimethylsulfoxid-Lösung, die 0,1 mmol/l des fluorogenen Substrates (7-Methoxycoumarin-4-yl)acetyl-Pro-Leu-Gly-Leu-3-(2',4'-dinitrophenyl)-L-2,3-diaminopropionyl-Ala-Arg-NH₂ (Bachem, Heidelberg, Deutschland) enthält, inkubiert und die Enzymreaktion fluoreszenzspektroskopisch verfolgt (328 nm (ex) / 393 nm(em)). Die Fluoreszenzmesung erfolgt für 15 Minuten. Die initiale Geschwindigkeit der enzymatischen Reaktion wird ohne Ihnibitorzugabe ermittelt und als 100% Aktivität definiert.

Die eingesetzten Verbindungen waren unfraktioniertes (UF) Heparin mit einem Molekulargewicht von etwa 15000 Dalton und einer 3000 Dalton (LMW) Heparin-Fraktion (beide erhältlich bei Sigma) und Enoxaparin. Getestet wurden die MMP-Aktivitäten von MMP-2 und MMP-8.

Die in Tabelle 5 aufgeführten Inhibitionen wurden bei einer Konzentration von jeweils 1µg/ml der eingesetzten Verbindungen ermittelt und mit einer Kontrolle ohne Inhibitor (100%) verglichen.

**Tabelle 5**

| Verbindung | MMP-2 Inhibition (%) | MMP-8 Inhibition (%) |
|---|---|---|
| UF-Heparin | 41 | 1 |
| LMW-Heparin | 27 | 8 |
| Enoxaparin | 70 | 28 |

## Patentansprüche

1. Verwendung von niedermolekularen Heparin mit einem durchschnittlichen Molekulargewicht von etwa 3000 bis etwa 10000 aus der Reihe Enoxaparin, Nardroparin, Dalteparin, Certroparin, Parnaparin, Reviparin, Ardeparin/RD Heparin/RDH oder Tinzaparin oder eine Mischung derselben
zur Herstellung von Arzneimitteln zur Prophylaxe und Therapie von Erkrankungen, an deren Verlauf eine verstärkte Aktivität mindestens einer der Matrix-Metalloproteinasen neutrophilen Kollagenase, Aggrecanase, hADAMTS1 und Gelatinase A beteiligt ist aus der Reihe Osteoarthrosen, Spondylosen, Knorpelschwund nach Gelenktrauma oder längerer Gelenksruhigstellung nach Meniskus- oder Patellaverletzungen oder Bänderrissen oder eine Erkrankung des Bindegewebes wie Kollagenosen, Wundheilungsstörungen, oder Periodontalerkrankungen.

2. Verwendung gemäß Anspruch 1, **dadurch gekennzeichnet, dass** als niedermolekulares Heparin Enoxaparin eingesetzt wird.

3. Verwendung gemäß der Ansprüche 1 oder 2, **dadurch gekennzeichnet, dass** die Applikation des niedermolekularen Heparins durch subkutane, intraartikuläre, intraperitoneale oder intravenöse Injektion, bevorzugt durch intraartikuläre Injektion, erfolgt.

4. Verwendung gemäß Anspruch 3, **dadurch gekennzeichnet, dass** die Dosis des niedermolekularen Heparins in der Injektionslösung von etwa 5 µg bis etwa 200 mg, vorzugsweise von etwa 10 µg bis 40 mg, beträgt.

5. Verwendung gemäß Anspruch 3, **dadurch gekennzeichnet, dass** Tagesdosen an Enoxaparin von etwa 10 µg bis 500 mg, bevorzugt etwa 20 mg bis 100 mg eingesetzt werden.

6. Verwendung gemäß der Ansprüche 1 oder 2, **dadurch gekennzeichnet, dass** die Applikation des niedermolekularen Heparins durch rektale, orale, inhalative oder transdermale Applikation erfolgt.

7. Verwendung von ADAMTS1 zur Herstellung eines Testkits zur Bestimmung eines Inhibitors für die Aggrecanase, **dadurch gekennzeichnet, dass** man ADAMTS1, ein Substrat und einen Inhibitor miteinander inkubiert und die durch die Aggrecanase-Aktivität generierten Neoepitope bestimmt.

## Claims

1. The use of low molecular weight heparin with an average molecular weight of about 3 000 to about 10 000 chosen from among enoxaparin, nardoparin, dalteparin, certroparin, parnaparin, reviparin, ardeparin/RD heparin/RDH or tinzaparin or a mixture thereof for producing pharmaceuticals for the prophylaxis and therapy of disorders in whose course an enhanced activity of at least one of the matrix metalloproteinases neutrophil collagenase, aggrecanase, hADAMTS1 and gelatinase A is involved, chosen from among osteoarthroses, spondyloses, chondrolysis after joint trauma or prolonged joint immobilization after meniscus or patellar injuries or ligament tears or a disorder of connective tissue such as collagenoses, wound healing disturbances, or periodontal disorders.

2. The use as claimed in claim 1, wherein enoxaparin is employed as low molecular weight heparin.

3. The use as claimed in claims 1 or 2, wherein the administration of the low molecular weight heparin takes place by subcutaneous, intraarticular, intraperitoneal or intravenous injection, preferably by intraarticular injection.

4. The use as claimed in claim 3, wherein the dose of the low molecular weight heparin in the solution for injection is from about 5 µg to about 200 mg, preferably from about 10 µg to 40 mg.

5. The use as claimed in claim 3, wherein daily doses of enoxaparin of about 10 µg to 500 mg, preferably about 20 mg to 100 mg, are employed.

6. The use as claimed in claims 1 or 2, wherein the administration of the low molecular weight heparin takes place by rectal, oral, inhalational or transdermal administration.

7. The use of ADAMTS1 for producing a test kit for the determination of an inhibitor for aggrecanase, which comprises incubating ADAMTS1, a substrate and an inhibitor together, and determining the neoepitopes generated by the aggrecanase activity:

## Revendications

1. Utilisation d'héparine de faible masse moléculaire, ayant une masse moléculaire moyenne d'environ 3 000 à environ 10 000, choisie dans l'ensemble constitué par l'énoxaparine, la nardroparine, la daltéparine, la certroparine, la parnaparine, la réviparine, l'ardéparine/RD héparine/RDH et la tinzaparine ou d'un mélange de celles-ci,
pour la fabrication de médicaments destinés à la prophylaxie et au traitement thérapeutique de maladies au cours desquelles est impliquée une activité accrue d'au moins l'une des métalloprotéases matricielles collagénase neutrophile, aggrécanase, hADAMTS1 et gélatinase A, choisies dans l'ensemble comprenant les ostéoarthroses, les spondyloses; la chondroporose après traumatisme articulaire ou relativement longue immobilisation d'articulations après blessures du ménisque ou de la rotule ou déchirures des ligaments ou une maladie du tissu conjonctif telle que les collagénoses, les troubles de la cicatrisation ou des maladies périodontaires.

2. Utilisation selon la revendication 1, **caractérisée en ce que** l'énoxaparine est utilisée en tant qu'héparine de faible masse moléculaire.

3. Utilisation selon la revendication 1 ou 2, **caractérisée en ce que** l'administration de l'héparine de faible masse moléculaire s'effectue par injection sous-cutanée, intra-articulaire, intrapéritonéale ou intraveineuse, de préférence par injection intra-articulaire.

4. Utilisation selon la revendication 3, **caractérisée en ce que** la dose de l'héparine de faible masse moléculaire dans la solution injectable va d'environ 5 *µ*g à environ 200 mg, de préférence d'environ 10 *µ*g à 40 mg.

5. Utilisation selon la revendication 3, **caractérisée en ce qu'**on utilise des doses journalières d'énoxaparine d'environ 10 *µ*g à 500 mg, de préférence d'environ 20 mg à 100 mg.

6. Utilisation selon la revendication 1 ou 2, **caractérisée en ce que** l'administration de l'héparine de faible masse moléculaire s'effectue par administration rectale, orale, par inhalation ou par application transdermique.

7. Utilisation d'ADAMTS1 pour la fabrication d'un nécessaire d'essai destiné à la détermination d'un inhibiteur de l'aggrécanase, **caractérisée en ce qu'**on met à incuber ensemble de l'ADAMTS1, un substrat et un inhibiteur et on détermine les néoépitopes engendrés par l'activité aggrécanase.
